# FASCICULE DE BREVET EUROPEEN

(11) **EP 2 629 751 B1**
(45) Date de publication et mention de la délivrance du brevet: **07.03.2018**
(21) Numéro de dépôt: 11779736.5
(22) Date de dépôt: 10.10.2011
(51) Int. Cl.: A61K 36/185, A61K 8/97, A61K 36/86, A61Q 19/00, A61P 15/02

(54) **UN PRODUIT NATUREL PERMETTANT DE RESSERRER TEMPORAIREMENT LES MUQUEUSES DU VAGIN**
NATURPRODUKT ZUR VORÜBERGEHENDEN VERENGUNG DER VAGINALEN SCHLEIMHÄUTE
NATURAL PRODUCT TEMPORARILY TIGHTENING THE MUCOUS MEMBRANES OF THE VAGINA

(30) Priorité: 20.10.2010 FR 1004113
(43) Date de publication de la demande: 28.08.2013
(73) Titulaire: Laaroussi, Iallaizana, 93800 Epinay-sur-Seine (FR)
(72) Inventeur: Laaroussi, Iallaizana, 93800 Epinay-sur-Seine (FR)
(74) Mandataire: Gevers & Orès
(86) Numéro de dépôt international: PCT/FR2011/000547
(87) Numéro de publication internationale: WO 2012/052627

(56) Documents cités:
- NAWWAR M A M ET AL: "Gall polyphenolics of Tamarix aphylla", PHYTOCHEMISTRY, vol. 36, no. 4, 1 juillet 1994 (1994-07-01), pages 1035-1037, XP026631583, PERGAMON PRESS, GB ISSN: 0031-9422, DOI: 10.1016/S0031-9422(00)90486-2 [extrait le 1994-07-01]
- NAWWAR ET AL: "Tamarixellagic acid, an ellagitannin from the galls of Tamarix aphylla", PHYTOCHEMISTRY, vol. 35, no. 5, 1 mars 1994 (1994-03-01), pages 1349-1354, XP022320098, PERGAMON PRESS, GB ISSN: 0031-9422
- ISHAK M S ET AL: "TANNINS AND POLY PHENOLICS OF THE GALLS OF TAMARIX-APHYLLA-D", PLANTA MEDICA, vol. 21, no. 3, 1 janvier 1972 (1972-01-01), pages 246-253, XP009150436, THIEME VERLAG, DE ISSN: 0032-0943
- HAMMICHE V ET AL: "Traditional medicine in Central Sahara: Pharmacopoeia of Tassili N'ajjer", JOURNAL OF ETHNOPHARMACOLOGY, vol. 105, no. 3, 24 mai 2006 (2006-05-24), pages 358-367, XP025085896, ELSEVIER SCIENTIFIC PUBLISHERS LTD, IE ISSN: 0378-8741, DOI: 10.1016/J.JEP.2005.11.028 [extrait le 2006-05-24]
- "Tamarix aphylla - Athel tamarisk -Usages médicaux", Plantes d'Avenir, 2010, XP002652065, Extrait de l'Internet: URL:http://plantes-davenir.loncletom.fr/pl ants/tamarix-aphylla/ [extrait le 2011-07-20]

## Description

La présente invention se rapporte au domaine gynécologique et en particulier dans le domaine cosmétique.

Le poudre de les galles de tamarix articulata destinées au traitement de la forme du vagin notamment les femmes qui cherchent une solution bio pour resserrer le vagin.

Il existe des interventions chirurgicales qui consistent à rétrécir le vagin par une série de sutures parcourant circulairement les pavois vagin.

Suite à un accouchement, au vieillissement des tissus ou à une mauvaise qualité musculaire, certaines femmes présentent un vagin plus large, ainsi qu'un introït béant.

Ceci limite fortement les frottements du pénis sur les parois vaginales dont l'innervation sensitive est aussi altérée, ce qui peut atténuer la satisfaction et le plaisir sexuels.

Donc, l'intervention a pour but de redonner au vagin un diamètre optimal lui permettant ainsi de retrouver sa tonicité, son élasticité et donc d'améliorer aussi sa fonction lors de la satisfaction sexuelle. Souvent, on corrige une entrée vaginale devenue trop béante, redonnant ainsi une nouvelle jeunesse périnéale, comme avant l'accouchement.

Pour les femmes qui ne voulait pas subir des interventions chirurgicales pour rétrécir le vagin. Ce produit naturel végétal, les galles de tamarix, non toxique tiré de la gale de tamarix articulata , permettre de resserrer temporairement la muqueuses avant chaque rapport sexuel.

Pour qui : Pour les femmes qui ont eu un ou plusieurs accouchements; pour les femmes ménopausées; pour celles qui présentent une mauvaise qualité musculaire.

Réduire en poudre les galles de tamarix articulata et mélangé avec pinte d'eau et faire avec la toilette intime.

Résultat vagin serrer ou petit, pénétration difficile, sensation de faire l'amour pour la première fois.

Depuis des siècles les femmes dans les pays du nord d'Afrique et en Asie connaissent l'importance de tamarix articulata. Elles ont utilisées les feuilles et les galles de cet arbre comme produit dans différente recettes notamment la médecine traditionnel et le cosmétique.

Quelle que femmes sahraouies « Sahara occidental » ont bien gardé le secret de l'efficacité de cet arbre comme produit qui resserre aussi le muqueuses du vagin, et ce secret a transmet par des familles de génération a génération.

**La classification de cet arbre :**

| | |
|---|---|
| **Règne** | (Végétal) |
| **Embranchement:** | spermatophytes |
| **Sous Embranchement :** | Angiospermes |
| **Classe:** | Dicotylédones |
| **Ordre:** | Violales |
| **Famille:** | Tamaricaceae |
| **Genre:** | Tamarix |
| **Nom Latin:** | *Tamarix articulata*(Vahl. (Vahl.) |
| **Synonymes:** | Tamarix aphylla ((L.) H. Karst., 1882) |

Arbre de tamarix articulata atteint 10 m de hauteur et 40 à 50 cm de diamètre: trés polymorphe, il est Indifférent aux qualités du sol et il résiste aux mutations des paysans. La floraison se produit en automne. Le fruit est une capsule pyriforme contenant 20 à 30 graines surmontées d'une aigrette.
les tanin végétaux sont des substances dans certaines parties des végétaux. Ce sont par définition des composés poly phénoliques qui présentent, malgré la différence de leur structure, un ensemble de caractères communs:
- ils s'associent aux protéines et aux polyols de masses moléculaires élevées.
- ils précipitent les matières colorantes basiques, la plupart des alcaloïdes et un grand nombre de métaux lourds.

Ces différentes propriétés sont mises à profit dans plusieurs domaines d'application : tannerie, adhésifs, encres, résines, etc.

Les tanins se divisent en deux grandes catégories : les tanins hydrolysables (ou gallique) qui ont une structure de polyester ; au contact d'un acide dilué ou d'un enzyme (la tannase de l'Aspergillus) ils s'hydrolysent en donnant naissance à un sucre, du glucose, et à des acides carboxyphénoliques, et les tanins condensés (ou catéchiques) contiennent uniquement des groupes phénoliques liés directement les uns aux autres par le carbone. ils sont généralement constitués par la condensation de flavanols (catéchine) et de flavandiol (leucoanthacyanine) sous l'effet d'un acide dilué se polymérisent et donne naissance à un produit rouge insoluble et amorphe appelé phlobaphène.
les galle de tamarix articulata sont produites par la piqure d'un acarien du groupe phytoptes (eriophes thaix) sur les fleurs de tamarix articulata . Les peuplements de tamarix articulata sont particulièrement abondants dans le sud du Maroc. Les galles, récoltées de fin septembre à début novembre, sont d'une richesse exceptionnelle en tanin. Elles renferment généralement 50% à 60% de substances tannantes.

L'invention porte sur l'utilisation d'une composition cosmétique applicable sur les muqueuses du vagin, pour resserrer temporairement les muqueuses du vagin, caractérisée en ce qu'elle contient une quantité efficace d'un extrait de galles de Tamarix articulata dans un milieu physiologiquement acceptable. Le procédé de préparation de cette composition consiste à sécher les galles de tamarix articulata, à les broyer, puis à les réduire en poudre. Cette poudre est ensuite mélangée à une quantité d'eau suffisante pour obtenir une suspension.

On utilise la proposition obtenue par ce procédé pour préparer un produit cosmétique destiné à être appliqué préalablement à un rapport sexuel sur les muqueuses du vagin afin de resserre temporairement ces muqueuses.

## Revendications

1. Utilisation d'une composition cosmétique applicable sur les muqueuses du vagin, pour resserrer temporairement les muqueuses du vagin, **caractérisée en ce qu'**elle contient une quantité efficace d'un extrait de galles de *Tamarix articulata* dans un milieu physiologiquement acceptable.

2. Utilisation selon la revendication 1, **caractérisée en ce que** les galles de *Tamarix articulata* sont séchées puis broyées, et réduites en poudre, puis mélangées avec de l'eau pour obtenir une suspension de la poudre de *Tamarix articulata* dans l'eau.

3. Utilisation selon la revendication 1 ou 2, **caractérisée en ce que** la composition est appliquée préalablement à un rapport sexuel sur les muqueuses du vagin, afin de resserrer temporairement ces muqueuses.

## Patentansprüche

1. Verwendung einer kosmetischen Zusammensetzung, die auf die Schleimhäute der Vagina auftragbar ist, um die Schleimhäute der Vagina vorübergehend zu verengen, **dadurch gekennzeichnet, dass** sie eine wirksame Menge eines Gallapfelextrakts von *Tamarix articulata* in einem physiologisch akzeptablen Milieu enthält.

2. Verwendung nach Anspruch 1, **dadurch gekennzeichnet, dass** die Galläpfel von *Tamarix articulata* getrocknet und danach zerkleinert, danach zu Pulver verarbeitet, danach mit Wasser gemischt werden, um eine Suspension des Pulvers von *Tamarix articulata* in Wasser zu erhalten.

3. Verwendung nach Anspruch 1 oder 2, **dadurch gekennzeichnet, dass** die Zusammensetzung vor einem Geschlechtsakt auf die Schleimhäute der Vagina aufgetragen wird, um diese Schleimhäute vorübergehend zu verengen.

## Claims

1. Use of a cosmetic composition applicable to the mucous membranes of the vagina, for temporarily tightening the mucous membranes of the vagina, **characterized in that** it contains an effective amount of a gall extract of *Tamarix articulata* in a physiologically acceptable medium.

2. Use according to claim 1, **characterized in that** the *tamarix articulata* galls are dried, crushed, reduced to powder, and then mixed with water to obtain a suspension of *tamarix articulata* powder in water.

3. Use according to claim 1 or 2, **characterized in that** the composition is applied prior to sexual intercourse on the mucous membranes of the vagina to temporarily tighten these mucous membranes.
